## Europäisches Patentamt

## European Patent Office

## Office européen des brevets

(11) Numéro de publication: **0 150 152 B1**

(12) **FASCICULE DE BREVET EUROPEEN**

(45) Date de publication du fascicule du brevet:
**07.01.88**

(21) Numéro de dépôt: **85400096.5**

(22) Date de dépôt: **21.01.85**

(51) Int. Cl.⁴: $C\,09\,B\,47/067$, $C\,07\,D\,487/22$ // $G09F9/30$

(54) **Procédé de préparation de diphtalocyanines métalliques, produits obtenus et leur application à titre d'agents électroactifs.**

(30) Priorité: **24.01.84 FR 8401164**

(43) Date de publication de la demande:
**31.07.85 Bulletin 85/31**

(45) Mention de la délivrance du brevet:
**07.01.88 Bulletin 88/1**

(84) Etats contractants désignés:
**CH DE GB IT LI**

(56) Documents cités:
**EP - A - 0 032 256**
**FR - A - 799 901**

**CHEMICAL ABSTRACTS, vol. 68, no. 17, 22 avril 1968, page 7569, no. 78403g, Columbus, Ohio, US; L.P. SHKLOVER et al.: "New phthalocyanines of rare earths"**
**CHEMICAL ABSTRACTS, vol. 72, no. 14, 6 avril 1970, page 611, no. 74210b, Columbus, Ohio, US; P.N. MOSKALEV et al.: "Synthesis and some properties of yttrium and erbium diphthalocyanines"**

(73) Titulaire: **Clarisse, Christian, 7, Avenue de Normandie, F-22300 Lannion (FR)**
Titulaire: **Riou née Meston, Marie-Thérèse, 8, rue Etienne d'Orves, F-22700 Perros Guirec (FR)**
Titulaire: **Auregan, Marcel, 3, Rue Charles Colvez, F-22300 Lannion (FR)**

(72) Inventeur: **Clarisse, Christian, 7, Avenue de Normandie, F-22300 Lannion (FR)**
Inventeur: **Riou née Meston, Marie-Thérèse, 8, rue Etienne d'Orves, F-22700 Perros Guirec (FR)**
Inventeur: **Auregan, Marcel, 3, Rue Charles Colvez, F-22300 Lannion (FR)**

(74) Mandataire: **Warcoin, Jacques et al, Cabinet Régimbeau 26, avenue Kléber, F-75116 Paris (FR)**

ACTORUM AG

## Description

La présente invention concerne un procédé de préparation de diphtalocyanine métallique utilisable notamment comme composant électrochrome.

Les diphtalocyanines de métaux di ou trivalents tels que l'yttrium ou les lanthanides sont connues pour présenter des propriétés électrochromes.

On dit d'un composé qu'il présente des propriétés électrochromes si ce composé est susceptible de changer de couleur lorsqu'on modifie les caractéristiques du potentiel électrique qu'on lui applique.

La diphtalocyanine de lutécium qui est de loin le composé le plus étudié dans ce domaine peut, lorsqu'elle est soumise dans une cellule électrolytique appropriée à des variations de potentiel, prendre n'importe quel degré d'oxydation compris entre (−2) et (+2) et correspondant aux couleurs suivantes: violet (−2), bleu (−1), vert (0), jaune (+1), rouge (+2).

On conçoit, bien entendu, l'intérêt de ce type de composé dans les dispositifs de visualisation et d'affichage colorés.

Bien que l'on ait proposé de nombreux procédés de préparation de ces composés, l'expérience montre qu'il est impossible d'obtenir, de la part des laboratoires de synthèse organique des diphtalocyanines en particulier, la diphtalocyanine de lutécium suffisamment purs pour être utlisée directement sans purification physique préalable telle que la sublimation, les produits livrés ayant toujours des couleurs et des propriétés variables selon les lots.

La disparité dans les propriétés de ces produits provient des procédés de synthèse mis en œuvre qui conduisent à la formation de nombreux sous-produits. La présence de ces sous-produits nécessite l'utilisation de méthodes de purification fort complexes parmi lesquelles les extractions successives, la sublimation fractionnée ou la chromatographie. Ces méthodes analytiques ne sont pas adaptées à la préparation de diphtalocyanine de lutécium en quantité importante. Il est donc préférable d'obtenir un produit pur dès la fin de la synthèse, directement utilisable pour réaliser des dispositifs électrochromes, ce qui est le but de la présente invention.

En outre, les procédés de la technique antérieure conduisent à des rendements très faibles ce qui est particulièrement gênant compte tenu de coût élevé des matières de départ.

La plupart des synthèses proposées dans la technique antérieure pour les diphtalocyanines métalliques consistent à chauffer un dérivé du métal en cause avec un phtalonitrile ou bien avec la phtalocyanine libre.

De tels procédés sont décrits notamment dans l'article de P.N.Moskalev et I.S.Kirin (Russian Journal of Inorganic Chemistry 15, 1, 1970) dans l'article de A.G.Mac Kay et coll (Aust. J. Chem. 1974, 27, 955–64) ou bien dans le brevet anglais 2063903.

Dans les deux articles cités précédemment, on constate que si l'on s'est beaucoup préoccupé des caractéristiques et des structures des produits préparés, les procédés mis en œuvre pour la synthèse de ces composés sont peu détaillés, en tout état de cause ils conduisent à des mélanges fort complexes dont les propriétés électrochromes sont parfois très délicates à mettre en évidence.

Il convient de remarquer que les procédés de la technique antérieure consistent essentiellement, après avoir mélangé les produits de départ, à chauffer le mélange et, après des temps de chauffage très variables ou indéterminés selon les auteurs, à prélever le mélange de réaction et à tenter d'en extraire le composé recherché.

La présente invention repose sur une analyse fine de la réaction et sur la mise en évidence de deux étapes distinctes dans la synthèse des diphtalocyanines métalliques.

En effet, il apparaît que la synthèse des diphtalocyanines à partir de phtalonitrile commence par une étape endothermique au cours de laquelle il est impératif de maintenir la température du milieu réactionnel au-dessous d'une température maximum qui sera appelée ci-après «température critique d'endothermicité».

Au cours de cette première étape, il se forme, essentiellement bien que non exclusivement, un composé intermédiaire indispensable dans la synthèse de la diphtalocyanine de lutécium, vraisemblablement une monophtalocyanine substituée ou non.

Lorsque cette étape endothermique est complètement terminée, on assiste à une montée en température qui correspond à une seconde étape exothermique. Cette réaction exothermique commence lentement au cours de la première étape, mais elle est masquée par la réaction endothermique beaucoup plus importante. Dans cette seconde étape, il convient là aussi de maintenir la température réactionnelle inférieure à une température maxima qui correspond à la température de décomposition des produits de départ et des produits formés.

Cette seconde étape correspond à la formation de la diphtalocyanine métallique proprement dite. La diphtalocyanine métallique formée est de couleur verte.

C'est pourquoi la présente invention concerne un procédé de préparation de diphtalocyanine de lanthanide caractérisé en ce que

a) on chauffe un mélange de dérivé de lanthanide et de phtalonitrile en maintenant la température de réaction inférieure à la température critique d'endothermicité puis,

b) lors de l'apparition d'une exothermicité, on maintient la température à une température inférieure à la température de décomposition des produits en réaction en particulier des produits de départ et de produits formés.

Il convient de remarquer que le terme lanthanide utilisé dans la présente description pourra désigner non seulement les lanthanides vrais (du lanthane au lutécium) mais également les métaux associés tels que l'yttrium dont les caractéristiques électroniques sont voisines de celles des lanthanides.

Parmi les dérivés de lanthanides utilisables, il faut citer plus particulièrement les dérivés organiques des lanthanides notamment les acétates.

Comme on l'a indiqué précédemment, la température critique d'endothermicité est fonction des produits de départ mis en œuvre et dans le cas du lutécium sous forme d'acétate cette température critique d'endothermicité est de 305 °C, pour ce qui concerne la seconde température de décomposition elle est bien entendu fonction des produits de départ là aussi dans le cas de l'acétate de lutécium, cette température de décomposition est de l'ordre de 320 °C.

Plus précisément pour le lutécium la température de la première étape sera de préférence comprise entre 290 et 305 °C. La durée de cette étape dépend de la température de la réaction, la fin de la réaction est caractérisée par l'apparition d'une réaction exothermique caractéristique de la formation de la diphthalocyanine. La température de la seconde étape est de préférence comprise entre 305 et 320 °C, pour éviter la formation massive de monophtalocyanine de lutécium de couleur bleue. Là aussi, la durée de cette étape dépend notamment de la température réactionnelle.

A titre d'exemple, on peut indiquer un profil de température intéressant qui correspond à la réaction de l'acétate de lutécium sur l'orthophthalonitrile (rapport 1/10 moles);

a) température de consigne variant linéairement de 300 à 308 °C en 100 mn;

b) température de consigne constante 308 °C pendant 100 mn.

Dans le cadre de la présente description, on décrira essentiellement la préparation de diphtalocyanine de lutécium qui est le composé le plus intéressant, mais il est certain que ce procédé peut être facilement transposé à d'autres terres rares présentant également un intérêt comme Gd, Tb, Dy, Ho, Er, Tm et Yb pour lesquelles les températures de formation des diphtalocyanines ne recouvrent pas le domaine des températures critiques.

Pour les autres terres rares, on peut dans certains cas avoir des difficultés à mettre en œuvre la seconde étape réactionnelle car les températures critiques peuvent être facilement atteintes, auxquelles on ne modère pas la réaction de synthèse exothermique. Ceci s'accompagne d'une diminution de rendement et entraîne la formation de phtalocyanine libre.

La réaction est de préférence conduite à la pression atmosphérique par exemple en réacteur ouvert.

Il est intéressant de travailler sous un balayage gazeux en particulier en balayage de gaz inerte tel que l'argon, en effet, ce balayage favorise l'élimination de l'acide acétique formé ce qui déplace l'équilibre de la réaction vers la formation du produit désiré.

Bien entendu, les produits de départ doivent être aussi purs que possible, ainsi l'orthodiphtalonitrile sera recristallisé deux fois, et le dérivé de lanthanide sera synthétisé le plus pur possible.

Il est important, en outre, de maintenir une agitation du milieu réactionnel afin d'opérer en phase homogène car, à l'état fondu, les éléments de réaction ont des densités très différentes. Cette agitation améliore également le dégagement de l'acide acétique formé.

Le rapport molaire des composés mis en œuvre est de préférence compris entre 1/6 et 1/12, dérivé métallique/phtalonitrile, de préférence 1/10. On utilise en principe un excès de phtalonitrile (moins cher que le dérivé métallique) pour déplacer la réaction, mais ceci conduit rapidement à la formation de phtalocyanine libre, c'est pourquoi il n'est pas souhaitable d'utiliser de trop grand excès molaire de phtalonitrile.

Les résultats observés lors de la mise en œuvre du procédé selon la présente invention ont permis de mettre en évidence deux avantages considérables de la présente invention, à savoir:

– une simplification considérable dans les techniques de purification et,

– un rendement considérablement accru par rapport aux rendements observés avec les techniques antérieures.

En effet, grâce à un simple lavage au méthanol et au diméthylformamide du produit brut, on obtient la diphtalocyanine de lutécium pure avec un rendement d'environ 60%. Alors que dans la technique antérieure les rendements en produit pur sont bien inférieurs, et que la teneur en phtalocyanine libre indésirable est supérieure à celle obtenue dans la mise en œuvre de la présente invention.

La teneur en phtalocyanine libre du produit obtenu est suffisamment faible pour qu'il soit directement utilisable après de simples lavages.

Cette amélioration dans les rendements de la synthèse de diphtalocyanine est d'une importance considérable compte tenu des prix des matériaux mis en œuvre puisqu'en effet l'acétate de lutécium utilisé comme produit de départ coûte environ 150000 francs 1983 le kg.

Les exemples ci-après sont destinés à mettre en évidence d'autres avantages et caractéristiques du procédé selon la présente invention.

Exemple 1

Synthèse de la diphtalocyanine de lutécium

On mélange au mortier environ 2 g d'acétate de lutécium déshydraté et de phtalonitrile recristallisé deux fois dans le rapport molaire 1 mole d'acétate de lutécium pour 10 moles de phtalonitrile.

On introduit ce mélange dans un tube ouvert placé verticalement dans un four. Ce mélange est agité en permanence. On porte la température du mélange à 304 °C pendant environ 1 h 30 min, puis, lorsque la température s'élève, on la maintient à 320 °C, cette température est maintenue pendant 1 h 30 min environ.

Le tube est alors sorti et refroidi à l'air libre. Le produit noir et friable formé au fond du tube est recueilli et broyé au mortier.

Ce produit est lavé à trois reprises avec du méthanol très pur, chaque lavage comprenant deux opérations à savoir agitation aux ultrasons puis centrifugation pour séparer les deux phases. Le produit est ensuite soumis à trois lavages avec le diméthylformamide selon le même procédé.

On termine enfin par deux rinçages au méthanol avec séchage à 100 °C.

Après séchage, le produit obtenu est caractérisé par spectroscopie infrarouge et uv-visibles.

On constate que le pourcentage de phtalocyanine libre caractérisé par spectroscopie infrarouge calculée par rapport à la diphtalocyanine est inférieure à 5%, alors qu'il est de plusieurs dizaines de % dans les produits obtenus par les procédés de la technique antérieure.

Exemple 2

Cet exemple a permis de mettre en évidence l'importance de la température critique d'endothermicité qui est pour l'acétate de lutécium de 305 °C.

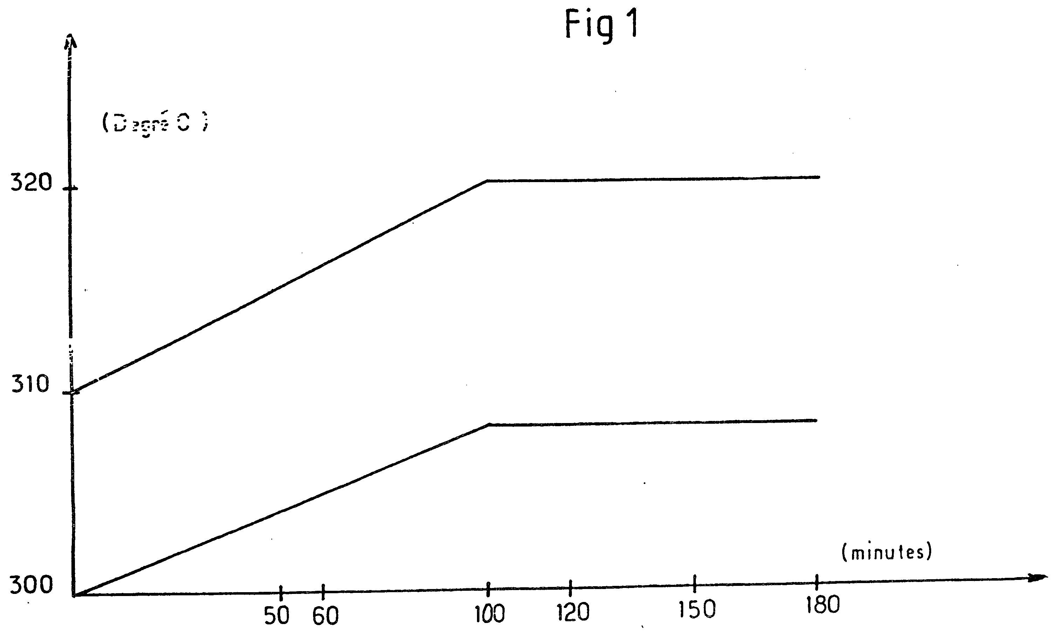

On a donc réalisé des essais comparatifs dans les conditions de l'exemple 1 en utilisant toutefois les 2 profils de température de consigne indiqués dans la figure ci-annexés.

Dans une première expérience, on a fait varier le point de consigne de la régulation linéairement de 300 °C à 308 °C durant la période exothermique.

Il s'agit là d'un profil de température conforme à celui du procédé selon la présente invention.

Dans une deuxième série d'expériences, on a fait varier le point de consigne de la température de 310 °C à 320 °C (c'est-à-dire au-dessus de la température critique d'endothermicité) en 100 mn et on l'a maintenu ensuite à 320 °C pendant 100 mn.

Dans les deux cas, les produits obtenus ont été lavés de la même façon avec les mêmes solvants.

Ces produits ont été caractérisés et les résultats sont rappelés dans le tableau ci-annexé.

Tableau

|  | 1er exemple | 2ème exemple |
|---|---|---|
| Solubilité dans le dichlorométhane | couleur verte soluble (40 mg/ 100 ml) solution verte | couleur bleue peu soluble ≤10 mg/ 100 ml solution bleue |
| Spectre uv-visible | 659 nm 1451 cm$^{-1}$ | 627 nm 1332 cm$^{-1}$ |
| Spectre I.R. | 1322 cm$^{-1}$ | 1074 cm$^{-1}$ 1063 cm$^{-1}$ |
| Electrochimie en solution (voltammétrie cyclique) | réaction parfaitement réversible conduisant aux différentes couleurs précitées | aucune réversibilité |
| Sublimation | se sublime à partir de 460° sans décomposition – film vert | ne se sublime pas du tout |

On constate à la lecture de ce tableau qu'une simple variation de 10° dans le profil de température de la réaction conduit à deux produits totalement différents. En fait, seul le procédé selon l'invention permet d'obtenir sans ambiguïté la diphtalocyanine de lutécium.

Revendications

1. Procédé préparation de diphtalocyanine de lanthanide ou d'ytterium, caractérisé en ce que:

a) on chauffe un mélange de dérivé de lanthanide ou d'yttrium et de phtalonitrile en maintenant la température de réaction inférieure à la température critique d'endothermicité puis,

b) lors de l'apparition d'une exothermicité, on maintient la température à une température inférieure à la température de décomposition des produits en réaction,

c) on récupère la diphtalocyanine de lanthanide ou d'yttrium obtenue.

2. Procédé selon la revendication 1, caractérisé en ce que le dérivé de lanthanide ou d'yttrium est un acétate.

3. Procédé selon la revendication 2, caractérisé en ce qu'il s'agit de l'acétate de lutécium, que la température critique d'endothermicité est de 305 °C et que la température de décomposition des produits en réaction est de 320 °C.

4. Procédé selon l'une des revendications 1 à 3, caractérisé en ce que le rapport molaire entre le dérivé de lanthanide ou d'yttrium et le phtalonitrile est compris entre 1/6 et 1/12.

5. Procédé selon l'une des revendications 1 à 4, caractérisé en ce que la réaction est conduite avec un balayage de gaz neutre, sous agitation permanente.

6. Procédé selon la revendication 5, caractérisé en ce que le balayage gazeux est un balayage d'argon.

7. Procédé selon l'une des revendications 1 à 6, caractérisé en ce que la réaction est conduite à la pression atmosphérique.

8. Procédé selon l'une des revendications 1 à 7, caractérisé en ce que la réaction est conduite en phase solide.

9. Procédé selon l'une des revendications 1 à 8, caractérisé en ce que la diphtalocyanine de lanthanide ou d'yttrium est récupérée après lavage du produit de réaction avec du méthanol et du diméthylformamide.

Patentansprüche

1. Verfahren zur Herstellung von Diphthalocyanin aus Lanthanid oder Yttrium, dadurch gekennzeichnet, dass:

a) man ein Gemisch aus Lanthanid- oder Yttriumderivat und Phthalonitril erhitzt und die Reaktionstemperatur unterhalb der kritischen Temperatur der Endothermizität hält, dann

b) man beim Auftreten von einer Exothermizität die Temperatur auf einer Temperatur unterhalb der Zersetzungstemperatur der Reaktionsprodukte hält,

c) man das Diphthalocyanin aus dem erhaltenen Lanthanid oder Yttrium wiedergewinnt.

2. Verfahren nach Anspruch 1, dadurch gekennzeichnet, dass das Derivat von Lanthanid oder Yttrium ein Acetat ist.

3. Verfahren nach Anspruch 2, dadurch gekennzeichnet, dass es sich um Lutetiumacetat handelt, dass die kritische Temperatur der Endothermizität bei 305 °C liegt und dass die Zersetzungstemperatur der Reaktionsprodukte bei 320 °C liegt.

4. Verfahren nach einem der Ansprüche 1 bis 3, dadurch gekennzeichnet, dass das Molverhältnis zwischen dem Derivat von Lanthanid oder Yttrium und dem Phthalonitril von 1/6 bis 1/12 umfasst.

5. Verfahren nach einem der Ansprüche 1 bis 4, dadurch gekennzeichnet, dass die Reaktion mit einer Spülung aus neutralem Gas, unter ständigem Umrühren, durchgeführt wird.

6. Verfahren nach Anspruch 5, dadurch gekennzeichnet, dass die Gasspülung eine Argonspülung ist.

7. Verfahren nach einem der Ansprüche 1 bis 6, dadurch gekennzeichnet, dass die Reaktion bei atmosphärischem Druck durchgeführt wird. ·

8. Verfahren nach einem der Ansprüche 1 bis 7, dadurch gekennzeichnet, dass die Reaktion in fester Phase durchgeführt wird.

9. Verfahren nach einem der Ansprüche 1 bis 8, dadurch gekennzeichnet, dass das Diphthalocyanin von Lanthanid oder Yttrium nach Waschen des Reaktionsproduktes mit Methanol und Dimethylformamid wiedergewonnen wird.

## Claims

1. Process for the preparation of lanthanide or yttrium diphthalocyanine, characterized in that:

a) a mixture of lanthanide or yttrium derivative and phthalonitrile is heated, the reaction temperature being kept below the critical temperature of endothermicity, then

b) when an exothermicity appears, the temperature is kept at a temperature below the decomposition temperature of the reactants,

c) the lanthanide or yttrium diphthalocyanine obtained is recovered.

2. Process as claimed in claim 1, characterized in that the lanthanide or yttrium derivative is an acetate.

3. Process as claimed in claim 2, characterized in that lutetium acetate is used, the critical temperature of endothermicity is 305 °C and the decomposition temperature of the reactants is 320 °C.

4. Process as claimed in one of claims 1 to 3, characterized in that the molar ratio of the lanthanide or yttrium derivative to phthalonitrile is between 1/6 and 1/12.

5. Process as claimed in one of claims 1 to 4, characterized in that the reaction is carried out under a stream of inert gas, with continuous stirring.

6. Process as claimed in claim 5, characterized in that the gas stream is a stream of argon.

7. Process as claimed in one of claims 1 to 6, characterized in that the reaction is carried out at atmospheric pressure.

8. Process as claimed in one of claims 1 to 7, characterized in that the reaction is carried out in solid phase.

9. Process as claimed in one of claims 1 to 8, characterized in that the lanthanide or yttrium diphthalocyanine is recovered after washing the reaction product with methanol and dimethylformamide.

Fig 1